# EUROPEAN PATENT APPLICATION

(11) **EP 4 729 079 A1**
(43) Date of publication of application: **22.04.2026**
(21) Application number: 24823722.4
(22) Date of filing: 13.06.2024
(51) Int. Cl.: A61L 27/18, A61L 27/34, A61L 27/58, C08L 67/04, C08L 101/16, A61L 27/10, A61L 27/54, B33Y 10/00, B33Y 80/00

(54) **IMPLANT COMPRISING BIODEGRADABLE POLYMER, MANUFACTURING METHOD THEREFOR, AND COMPOSITION THEREFOR**

(30) Priority: 14.06.2023 KR 20230076405
(71) Applicant: CJ Cheiljedang Corporation, Seoul 04560 (KR)
(72) Inventor: SIM, Eun Jung, Seoul 04560 (KR); LEE, Eun-Hye, Seoul 04560 (KR); SUNG, Hakyoung, Seoul 04560 (KR); YOON, Ki Chull, Seoul 04560 (KR); PARK, Won Ho, Seoul 04560 (KR); KANG, Chan, Seoul 04560 (KR); SONG, Jae Hwang, Seoul 04560 (KR); LEE, Su Jeong, Seoul 04560 (KR)
(74) Representative: Meissner Bolte Partnerschaft mbB
(86) International application number: PCT/KR2024/008122
(87) International publication number: WO 2024/258205

(57) **Abstract**

Provided is an implant comprising a first biodegradable polymer and a second biodegradable polymer, wherein the first biodegradable polymer comprises copolymerized polyhydroxyalkanoate (PHA), and the copolymerized PHA comprises, on the basis of the total weight of the copolymerized PHA, 0.1-50 wt% of a repeating unit derived from 4-hydroxybutyrate (4-HB).

## Description

### Technical Field

The present disclosure relates to an implant comprising a biodegradable polymer and to a process for manufacturing the same. More specifically, the present disclosure relates to an orthopedic implant comprising a biodegradable polymer and having improved osteogenic differentiation and osseointegration properties, and a process for manufacturing the same.

### Background Art

Biodegradable polymers are materials that are attracting attention in various fields such as medicine, agriculture, and environment by virtue of their unique decomposition characteristics. In particular, in the medical field, unlike metals and nondegradable polymers such as ceramics, they have the advantage of helping the body heal and eliminating the need for separate removal surgery after healing since they are broken down through body metabolism after completing their function.

Such biodegradable polymers are largely divided into natural biodegradable polymers and synthetic biodegradable polymers. Since natural biodegradable polymers are made of natural materials, they have excellent biological compatibility and biological adaptability, causing relatively little immune response in the body. However, since they have weaker physical strength and durability as compared with synthetic biodegradable polymers, appropriate materials must be used depending on the intended use.

Currently, polyglycolide (PGA), polylactide (PLLA), and polycaprolactone (PCL) are used as main materials for absorbable implants, and synthetic biodegradable polymers are most used. PLLA, which is the most commonly used, has high strength whereas it is weak against impact; thus, materials (PLGA or PLCL) copolymerized with PGA or PCL are often used. For example, Korean Laid-open Patent Publication No. 2022-0047788 discloses an implant made of a biodegradable composite material comprising PLLA, PGA, PCL, or a copolymer thereof.

Polyhydroxyalkanoate (PHA) is a bio-derived natural biodegradable polymer produced within microorganisms and is evaluated as a safe material since it decomposes in the human body and is not toxic. It can be applied to various medical materials such as fracture treatment, cardiovascular materials, artificial joints, and surgical tools by virtue of the above advantages. There are various types of PHA such as PHB, P3HB, P4HB, PHV, and PHH. Since each type has different characteristics, an appropriate material is selected depending on the application, or a copolymer such as P(3HB-4HB) may be prepared for use.

The development of absorbable implants for fracture treatment containing such a bio-derived polymer is expected to make an important contribution to enhancing the quality of fracture treatment, thereby shortening the patients' recovery period and reducing the burden during the rehabilitation process. Further, it is expected to contribute to reducing medical costs related to fracture treatment, which has become more important in an aging society.

### [Prior Art Document]

(Patent Document 1) Korean Laid-open Patent Publication No. 2022-0047788

### Disclosure of Invention

### Technical Problem

An object of the present disclosure is to provide an implant that comprises a biodegradable polymer, has strength and stability suitable for orthopedics, and has excellent osteogenic differentiation and osseointegration properties, a process for manufacturing the same, and a composition for the same.

### Solution to Problem

The present disclosure provides an implant that comprises a first biodegradable polymer and a second biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

In an embodiment, the implant may comprise a blend of the first biodegradable polymer and the second biodegradable polymer.

In another embodiment, the implant may comprise a core part and a sheath part, the sheath part may comprise the first biodegradable polymer, and the core part may comprise the second biodegradable polymer.

In another embodiment, the implant may comprise a core part and a sheath part, the sheath part and the core part each comprise a blend of the first biodegradable polymer and the second biodegradable polymer, and the content of the first biodegradable polymer contained in the sheath part based on the total weight of the blend contained in the sheath part may be greater than the content of the first biodegradable polymer contained in the core part based on the total weight of the blend contained in the core part.

In another embodiment, the implant comprises at least one intermediate part disposed between the core part and the sheath part, at least one intermediate part comprises a blend of the first biodegradable polymer and the second biodegradable polymer, the content of the first biodegradable polymer contained in the sheath part based on the total weight of the blend contained in the sheath part may be greater than the content of the first biodegradable polymer contained in the intermediate part based on the total weight of the blend contained in the intermediate part, and the content of the first biodegradable polymer contained in the intermediate part based on the total weight of the blend contained in the intermediate part may be greater than the content of the first biodegradable polymer contained in the core part based on the total weight of the blend contained in the core part.

In another embodiment, the implant may have a porous structure comprising pores with a size of 10 nm to 10 µm.

In another embodiment, the copolymeric polyhydroxyalkanoate (PHA) may further comprise a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

In another embodiment, the first biodegradable polymer may further comprise at least one polymer selected from the group consisting of poly(3-hydroxybutyrate) (P3HB), poly(4-hydroxybutyrate) (P4HB), poly(3-hydroxyhexanoate) (P3HH), poly(3-hydroxybutyrate-co-3-hydroxyhexanoate) (P3HB-3HH), poly(3-hydroxyoctanoate) (P3HO), and poly(3-hydroxybutyrate-co-3-hydroxyoctanoate) (P3HB-3HO).

In another embodiment, the second biodegradable polymer is one, or a mixture of two or more, selected from synthetic biodegradable polymers and natural biodegradable polymers excluding polyhydroxyalkanoate (PHA), the synthetic biodegradable polymer may comprise polylactic acid (PLA), polycaprolactone (PCL), polyglycolic acid (PGA), poly(lactic acid-co-caprolactone) (PLCL), poly(lactic acid-co-glycolic acid) (PLGA), polybutylene succinate (PBS), and poly(butylene adipate-co-terephthalate) (PBAT); and the natural biodegradable polymer may comprise starch, silk fibroin, chitosan, chitin, cellulose, collagen, and gelatin.

In another embodiment, the copolymeric polyhydroxyalkanoate (PHA) may be included in an amount of 10% by weight to 50% by weight based on the total weight of the first biodegradable polymer and the second biodegradable polymer.

In another embodiment, the implant may further comprise at least one additive selected from bioactive glass fiber or ceramic.

In another embodiment, the implant further comprises at least one drug for enhancing bone regeneration, and the drug may be coated on the surface of the implant, may be blended with the first biodegradable polymer and the second biodegradable polymer, or may be included in a concentration gradient from the surface to the inside of the implant.

In another embodiment, the implant may have a tensile strength of 40 MPa to 70 MPa and an elongation of 3.2% to 10%, as measured on specimens prepared with a diameter of 1.5 mm and a length of 50 mm.

In another embodiment, the implant may have a surface roughness with a depth of 1 µm to 10 µm, or a grid pattern, a wave pattern, or a stripe pattern with an interval of 1 µm to 10 µm.

In another embodiment, the implant, in a cytotoxicity test using an eluate, may exhibit a cell viability of 70% or more relative to the cell viability of 100% of an elution solvent control group.

In another embodiment, when the implant is implanted into a fractured femur tissue of a rat, the stiffness of the femur tissue harvested after 2 months from the implantation may be 120 N/mm or more.

In addition, the present disclosure provides a process for manufacturing an implant that comprises preparing a biodegradable polymer comprising a first biodegradable polymer and a second biodegradable polymer; and forming an implant using the biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

In an embodiment, the forming may be injection molding or 3D printing molding.

In another embodiment, the injection molding may be carried out under the conditions of an extrusion temperature of 100°C to 210°C, an injection temperature of 150°C to 200°C, and an annealing temperature of 80°C to 110°C.

In another embodiment, the 3D printing molding may be carried out under the conditions of a printing temperature of 150°C to 200°C, an injection speed of 200 mm/minute to 400 mm/minute, a pressure of 200 kPa to 400 kPa, and a printing duration time of 60 minutes or less.

In another embodiment, the process may further comprise, after the forming, at least one surface treatment step among imparting a roughness or a pattern to the surface through blasting, molding, or laser treatment; and imparting hydrophilicity to the surface through plasma treatment.

In addition, the present disclosure provides a composition for an implant that comprises a first biodegradable polymer and a second biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

### Advantageous Effects of Invention

As the implant according to the present disclosure comprises a first biodegradable polymer comprising a copolymeric polyhydroxyalkanoate (PHA) and a second biodegradable polymer, it exhibits appropriate strength and stability for orthopedic implants, for example, a material for hard tissues fixation such as pins, screws, and fixation plates, and has excellent osteogenic and osseointegration properties.

### Brief Description of Drawings

Fig. 1a shows an implant (11) according to an embodiment.
Fig. 1b shows an implant (12) according to another embodiment (100: core part, 300: sheath part).
Fig. 1c shows an implant (13) according to another embodiment (100: core part, 200: intermediate part; 300: sheath part).
Fig. 2 schematically shows the surface roughness of an implant.
Fig. 3 shows the results of a tensile test of an implant.
Fig. 4 shows the results of a cytotoxicity test of an implant.
Fig. 5 shows the results of WST-1 analysis of an implant.
Fig. 6 shows the results of an osteogenic differentiation induction test of an implant.
Fig. 7 shows an overview of a rat femur fracture test.
Fig. 8 shows the results of histopathological analysis of a rat femur fracture test.
Fig. 9 shows micro-CT 2D and 3D images of a rat femur fracture test.
Figs. 10a and 10b show the results of radiological analysis of rat femur samples in the 2^{nd} month.
Figs. 11a and 11b show the results of radiological analysis of rat femur samples in the 4^{th} month.
Figs. 12a and 12b show the results of biomechanical analysis of a rat femur fracture test.

### Best Mode for Carrying out the Invention

Hereinafter, the present disclosure will be described in detail with reference to the embodiments. The embodiments are not limited to those described below. Rather, they can be modified into various forms as long as the gist of the invention is not altered.

In the present specification, the term "comprising" is intended to specify a particular characteristic, region, step, process, element, and/or component. It does not exclude the presence or addition of any other characteristic, region, step, process, element and/or component, unless specifically stated to the contrary.

In the present specification, the terms first, second, and the like are used to describe various components. But the components should not be limited by the terms. The terms are used for the purpose of distinguishing one element from another.

In the numerical range that limits the size of components, physical properties, and the like described in the present specification, when a numerical range limited with the upper limit only and a numerical range limited with the lower limit only are separately exemplified, it should be understood that a numerical range combining these upper and lower limits is also encompassed in the exemplary scope.

### Biodegradable polymer

The implant according to the present disclosure comprises a biodegradable polymer. For example, it may comprise a bio-derived polymer.

Specifically, the implant may comprise two or more types of biodegradable polymers.

In an embodiment, the implant comprises a first biodegradable polymer and a second biodegradable polymer.

In another embodiment, the implant may comprise a blend of the first biodegradable polymer and the second biodegradable polymer.

The first biodegradable polymer and the second biodegradable polymer may be polymers different from each other.

The first biodegradable polymer and the second biodegradable polymer may each comprise one, two, or more types of biodegradable polymers.

In an embodiment, the first biodegradable polymer may be a polyhydroxyalkanoate (PHA).

The polyhydroxyalkanoate (PHA) may have physical properties similar to those of conventional petroleum-derived synthetic polymers such as polybutylene adipate terephthalate (PBAT), polybutylene succinate (PBS), polybutylene succinate terephthalate (PBST), and polybutylene succinate adipate (PBSA), exhibits excellent biodegradability, and is excellent in biocompatibility.

Specifically, the PHA is a natural polyester-based polymer that accumulates in microbial cells. It can ultimately be decomposed into carbon dioxide, water, and organic substances. In particular, since the PHA can be decomposed *in vivo,* an implant manufactured using a biodegradable polymer composition comprising the PHA can be used in the medical field as an absorbable medical device.

The PHA may be formed by an enzyme-catalyzed polymerization of one or more monomer repeat units in living cells.

In an embodiment, the first biodegradable polymer comprises a copolymeric polyhydroxyalkanoate (PHA).

For example, the first biodegradable polymer may comprise one, two, or more types of copolymeric polyhydroxyalkanoates (PHAs).

The copolymeric polyhydroxyalkanoate (PHA) may comprise a copolymer comprising two or more different repeat units with the different repeat units randomly distributed in the polymer chain.

In an embodiment, the copolymeric polyhydroxyalkanoate (PHA) may comprise a repeat unit derived from 4-hydroxybutyrate (4-HB).

In the present disclosure, it is important to adjust the content of the 4-HB repeat unit in the copolymeric PHA. That is, in order to achieve the physical properties desired in the present disclosure, in particular, to increase biodegradability *in vivo* and to achieve excellent physical properties, the content of the 4-HB repeat unit in the copolymeric PHA may be important.

The content of a repeat unit derived from the 4-hydroxybutyrate (4-HB) may be 0.1% by weight or more, 5% by weight or more, 10% by weight or more, 12% by weight or more, 13% by weight or more, 15% by weight or more, 17% by weight or more, 18% by weight or more, 20% by weight or more, or 25% by weight or more, and 50% by weight or less, 45% by weight or less, 43% by weight or less, 42% by weight or less, 40% by weight or less, or 35% by weight or less, based on the total weight of the copolymeric polyhydroxyalkanoate (PHA).

For example, the content of a repeat unit derived from the 4-hydroxybutyrate (4-HB) may be 0.1% by weight to 50% by weight, 1% by weight to 50% by weight, 2% by weight to 50% by weight, 3% by weight to 50% by weight, 5% by weight to 50% by weight, 10% by weight to 50% by weight, 1% by weight to 40% by weight, 1% by weight to 30% by weight, 1% by weight to 29% by weight, 1% by weight to 25% by weight, 1% by weight to 24% by weight, 2% by weight to 20% by weight, 2% by weight to 23% by weight, 3% by weight to 20% by weight, 3% by weight to 15% by weight, 4% by weight to 18% by weight, 5% by weight to 15% by weight, 8% by weight to 12% by weight, 9% by weight to 12% by weight, 15% by weight to 50% by weight, 20% by weight to 50% by weight, 25% by weight to 50% by weight, based on the total weight of the copolymeric polyhydroxyalkanoate (PHA), but it is not limited thereto.

In an embodiment, the copolymeric polyhydroxyalkanoate (PHA) may comprise a repeat unit derived from 4-hydroxybutyrate (4-HB) in an amount of 0.1% by weight to 50% by weight based on the total weight of the copolymeric polyhydroxyalkanoate (PHA).

More specifically, the copolymeric polyhydroxyalkanoate (PHA) may comprise a repeat unit derived from 4-hydroxybutyrate (4-HB) in an amount of 3% by weight to 20% by weight based on the total weight of the copolymeric polyhydroxyalkanoate (PHA).

As described above, the copolymeric PHA comprises one or more of a 4-HB repeat unit, and the content of the 4-HB repeat unit may be controlled to adjust the crystallinity of the copolymeric PHA. That is, the copolymeric PHA may be a polymer with controlled crystallinity.

The copolymeric PHA whose crystallinity is adjusted may be one in which its crystallinity and amorphousness are adjusted as the irregularities are increased in its molecular structure. Specifically, the types and ratios of the monomers or the types and/or contents of the isomers may be adjusted.

In addition, the copolymeric polyhydroxyalkanoate (PHA) may further comprise a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

Specifically, the copolymeric polyhydroxyalkanoate (PHA) may comprise at least one repeat unit selected from the group consisting of 3-hydroxybutyrate (3-HB), 3-hydroxypropionate (3-HP), 3-hydroxyvalerate (3-HV), 3-hydroxyhexanoate (3-HH), 4-hydroxyvalerate (4-HV), 5-hydroxyvalerate (5-HV), and 6-hydroxyhexanoate (6-HH).

More specifically, the copolymeric PHA may comprise a copolymer comprising a repeat unit derived from 3-HB and a repeat unit derived from 4-HB. For example, the copolymeric PHA may comprise poly(3-hydroxybutyrate-co-4-hydroxybutyrate) (3HB-co-4HB).

For example, the content of a repeat unit derived from the 3-HB may be 50% by weight or more, 55% by weight or more, 60% by weight or more, 64% by weight or more, 70% by weight or more, or 75% by weight or more, and 99.9% by weight or less, 99% by weight or less, 95% by weight or less, 90% by weight or less, 85% by weight or less, 80% by weight or less, or 75% by weight or less, based on the total weight of the copolymeric PHA.

In addition, the copolymeric PHA may comprise isomers. For example, the copolymeric PHA may comprise structural isomers, enantiomers, or geometric isomers. Specifically, the PHA may comprise structural isomers.

The copolymeric PHA may have a glass transition temperature (Tg) of, for example, -45°C to 80°C, -35°C to 80°C, -30°C to 80°C, -25°C to 75°C, -20°C to 70°C, - 35°C to 5°C, -25°C to 5°C, -35°C to 0°C, -25°C to 0°C, -30°C to -10°C, -35°C to -15°C, -35°C to -20°C, -20°C to 0°C, -15°C to 0°C, or -15°C to -5°C.

The crystallization temperature (Tc) of the copolymeric PHA, for example, may not be measured or may be, for example, 70°C to 120°C, 75°C to 120°C, 75°C to 115°C, 75°C to 110°C, or 90°C to 110°C.

The melting temperature (Tm) of the copolymeric PHA, for example, may not be measured or may be, for example, 100°C to 170°C, 110°C to 150°C, or 120°C to 140°C.

The copolymeric PHA may have a weight average molecular weight (Mw) of, for example, 10,000 g/mole to 1,200,000 g/mole. For example, the weight average molecular weight of the copolymeric PHA may be 50,000 g/mole to 1,200,000 g/mole, 100,000 g/mole to 1,200,000 g/mole, 50,000 g/mole to 1,000,000 g/mole, 100,000 g/mole to 1,000,000 g/mole, 100,000 g/mole to 900,000 g/mole, 200,000 g/mole to 1,200,000 g/mole, 250,000 g/mole to 1,150,000 g/mole, 300,000 g/mole to 1,100,000 g/mole, 350,000 g/mole to 1,000,000 g/mole, 350,000 g/mole to 950,000 g/mole, 100,000 g/mole to 900,000 g/mole, 200,000 g/mole to 800,000 g/mole, 200,000 g/mole to 700,000 g/mole, 250,000 g/mole to 650,000 g/mole, 200,000 g/mole to 400,000 g/mole, 300,000 g/mole to 800,000 g/mole, 300,000 g/mole to 600,000 g/mole, 400,000 g/mole to 800,000 g/mole, 500,000 g/mole to 1,200,000 g/mole, 500,000 g/mole to 1,000,000 g/mole 550,000 g/mole to 1,050,000 g/mole, 550,000 g/mole to 900,000 g/mole, or 600,000 g/mole to 900,000 g/mole.

In an embodiment, the copolymeric PHA may be a crystalline or semi-crystalline PHA. For example, the crystalline or semi-crystalline PHA may comprise a 4-HB repeat unit in an amount of, for example, 1% by weight to 25% by weight based on the total weight of the copolymeric PHA. In addition, the crystalline or semi-crystalline PHA may have a glass transition temperature (Tg) of, for example, -20°C to 0°C, a crystallization temperature (Tc) of, for example, 75°C to 115°C, and a melting temperature (Tm) of, for example, 110°C to 160°C.

In another embodiment, the first biodegradable polymer may further comprise at least one polymer selected from the group consisting of poly(3-hydroxybutyrate) (P3HB), poly(4-hydroxybutyrate) (P4HB), poly(3-hydroxyhexanoate) (P3HH), poly(3-hydroxybutyrate-co-3-hydroxyhexanoate) (P3HB-3HH), poly(3-hydroxyoctanoate) (P3HO), and poly(3-hydroxybutyrate-co-3-hydroxyoctanoate) (P3HB-3HO).

In another embodiment, the first biodegradable polymer may further comprise at least one type of copolymeric polyhydroxyalkanoate (PHA) with short or medium chains, in addition to the above.

The second biodegradable polymer is one, or a mixture of two or more, selected from synthetic biodegradable polymers and natural biodegradable polymers excluding polyhydroxyalkanoate (PHA).

Examples of the synthetic biodegradable polymer include polylactic acid (PLA), polycaprolactone (PCL), polyglycolic acid (PGA), poly(lactic acid-co-caprolactone) (PLCL), poly(lactic acid-co-glycolic acid) (PLGA), polybutylene succinate (PBS), and poly(butylene adipate-co-terephthalate) (PBAT).

Examples of the natural biodegradable polymer include starch, silk fibroin, chitosan, chitin, cellulose, collagen, and gelatin.

In an embodiment, the second biodegradable polymer may be a polylactide (PLA).

For example, the second biodegradable polymer may comprise one, two, or more types of polylactides (PLAs).

Specifically, the second biodegradable polymer may comprise a poly-L-lactide (PLLA), a poly-D-lactide (PDLA), a poly-DL-lactide (PDLLA), or a blend thereof.

A polylactide (PLA) has high strength with low flexibility, making it prone to fracture. However, as it is mixed with a copolymeric polyhydroxyalkanoate (PHA), flexibility is imparted. As a result, implant procedure stability, osteogenic property, and osseointegration property can be enhanced.

### Additive and drug

The implant may further comprise at least one additive and/or drug in addition to the biodegradable polymers.

In an embodiment, the implant may further comprise at least one additive selected from bioactive glass fiber or ceramic, but it is not limited thereto.

Examples of the bioactive ceramic include hydroxyapatite (HAP), wollastonite, tricalcium phosphate (TCP), dicalcium phosphate dihydrate (DCPD), tetracalcium phosphate (TTCP), octacalcium phosphate (OCP), and amorphous calcium phosphate (ACP).

In another embodiment, the implant may further comprise at least one drug for enhancing bone regeneration.

Examples of the drug include tricalcium phosphate (TCP), beta-tricalcium phosphate (β-TCP), hydroxyapatite-tricalcium phosphate (HTCP), deoxyribonucleotide triphosphate (dNTPs), bone morphogenetic proteins (BMPs), calcium (Ca), and magnesium (Mg).

In an embodiment, the drug may be coated on the surface of the implant.

In another embodiment, the drug may be blended with the first biodegradable polymer and the second biodegradable polymer.

In another embodiment, the drug may be included in a concentration gradient from the surface to the inside of the implant. As an example, the drug may be included such that its concentration is gradually decreased from the surface to the inside. In such a case, the amount of drug released may initially be rapid and then gradually decrease.

### Configuration of the implant

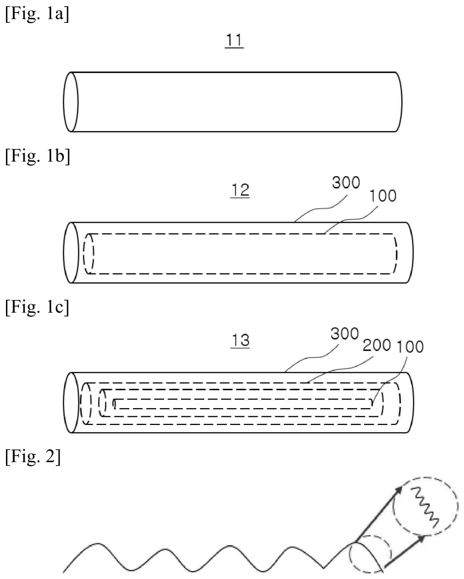

Fig. 1a shows an implant according to an embodiment.

Referring to Fig. 1a, the implant (11) may have a single-layer structure.

In an embodiment, the implant may comprise a blend of the first biodegradable polymer and the second biodegradable polymer.

The content of the first biodegradable polymer may be 0.1% by weight or more, 0.5% by weight or more, 1% by weight or more, 3% by weight or more, 5% by weight or more, 10% by weight or more, 15% by weight or more, 20% by weight or more, or 25% by weight or more, and may be 50% by weight or less, 45% by weight or less, 40% by weight or less, 35% by weight or less, 30% by weight or less, or 25% by weight or less, based on the total weight of the first biodegradable polymer and the second biodegradable polymer (i.e., the total weight of the blend).

Specifically, the content of the first biodegradable polymer may be 0.1% by weight to 50% by weight, 1% by weight to 50% by weight, 5% by weight to 50% by weight, 10% by weight to 50% by weight, 15% by weight to 50% by weight, 20% by weight to 50% by weight, 25% by weight to 50% by weight, 0.1% by weight to 40% by weight, 5% by weight to 40% by weight, 15% by weight to 40% by weight, 25% by weight to 40% by weight, 5% by weight to 35% by weight, 15% by weight to 35% by weight, or 25% by weight to 35% by weight, based on the total weight of the first biodegradable polymer and the second biodegradable polymer.

In addition, the content of the second biodegradable polymer may be 50% by weight or more, 55% by weight or more, 60% by weight or more, 64% by weight or more, 70% by weight or more, or 75% by weight or more, and 99.9% by weight or less, 99% by weight or less, 95% by weight or less, 90% by weight or less, 85% by weight or less, 80% by weight or less, or 75% by weight or less, specifically, 50% by weight to 90% by weight, based on the total weight of the first biodegradable polymer and the second biodegradable polymer.

For example, the weight ratio of the first biodegradable polymer and the second biodegradable polymer may be 0.1:99.9 to 50:50, 1:99 to 50:50, 5:95 to 50:50, 5:95 to 40:60, 10:90 to 50:50, 15:85 to 40:60, 20:80 to 40:60, or 25:75 to 40:60.

In an embodiment, the implant may comprise a blend of the first biodegradable polymer comprising the copolymeric polyhydroxyalkanoate (PHA) and the second biodegradable polymer.

The content of the copolymeric polyhydroxyalkanoate (PHA) may be 0.1% by weight or more, 0.5% by weight or more, 1% by weight or more, 3% by weight or more, 5% by weight or more, 10% by weight or more, 15% by weight or more, 20% by weight or more, or 25% by weight or more, and may be 50% by weight or less, 45% by weight or less, 40% by weight or less, 35% by weight or less, 30% by weight or less, or 25% by weight or less, based on the total weight of the first biodegradable polymer and the second biodegradable polymer.

Specifically, the content of the copolymeric polyhydroxyalkanoate (PHA) may be 0.1% by weight to 50% by weight, 1% by weight to 50% by weight, 5% by weight to 50% by weight, 10% by weight to 50% by weight, 15% by weight to 50% by weight, 20% by weight to 50% by weight, 25% by weight to 50% by weight, 0.1% by weight to 40% by weight, 5% by weight to 40% by weight, 15% by weight to 40% by weight, 25% by weight to 40% by weight, 5% by weight to 35% by weight, 15% by weight to 35% by weight, or 25% by weight to 35% by weight, based on the total weight of the first biodegradable polymer and the second biodegradable polymer.

For example, the copolymeric polyhydroxyalkanoate (PHA) may be included in an amount of 10% by weight to 50% by weight based on the total weight of the first biodegradable polymer and the second biodegradable polymer.

In an embodiment, the first biodegradable polymer may be a copolymeric polyhydroxyalkanoate (PHA), and the second biodegradable polymer may be a polylactide (PLA).

For example, the weight ratio of the copolymeric polyhydroxyalkanoate (PHA) and the polylactide (PLA) may be 0.1:99.9 to 50:50, 1:99 to 50:50, 5:95 to 50:50, 5:95 to 40:60, 10:90 to 50:50, 15:85 to 40:60, 20:80 to 40:60, or 25:75 to 40:60.

Fig. 1b shows an implant according to another embodiment.

Referring to Fig. 1b, the implant (12) according to another embodiment comprises a core part (100) and a sheath part (300).

In an embodiment, the sheath part may comprise the first biodegradable polymer, and the core part may comprise the second biodegradable polymer.

In another embodiment, the core part and the sheath part each comprise a blend of the first biodegradable polymer and the second biodegradable polymer, and the content of the first biodegradable polymer contained in the sheath part based on the total weight of the blend contained in the sheath part may be greater than the content of the first biodegradable polymer contained in the core part based on the total weight of the blend contained in the core part.

In another embodiment, the core part and the sheath part each comprise the first biodegradable polymer, the second biodegradable polymer, or a blend thereof, and the weight ratio of the first biodegradable polymer to the second biodegradable polymer contained in the sheath part may be greater than the weight ratio of the first biodegradable polymer to the second biodegradable polymer contained in the core part.

The size and thickness of the core part and the sheath part may vary depending on the content ratio of the first biodegradable polymer and the second biodegradable polymer contained in the implant.

Fig. 1c shows an implant according to another embodiment.

Referring to Fig. 1c, the implant (13) according to another embodiment comprises a core part (100), a sheath part (300), and at least one intermediate part (200) interposed between them.

For example, the sheath part may have the highest content of the first biodegradable polymer, the core part may have the highest content of the second biodegradable polymer, and at least one intermediate part may have a higher content of the first biodegradable polymer as it is closer to the sheath part and may have a higher content of the second biodegradable polymer as it is closer to the core part.

In an embodiment, the sheath part may comprise the first biodegradable polymer, and the core part may comprise the second biodegradable polymer.

In another embodiment, the core part and the sheath part each comprise a blend of the first biodegradable polymer and the second biodegradable polymer, and the content of the first biodegradable polymer contained in the sheath part based on the total weight of the blend contained in the sheath part may be greater than the content of the first biodegradable polymer contained in the core part based on the total weight of the blend contained in the core part.

The implant comprises at least one intermediate part disposed between the core part and the sheath part, at least one intermediate part comprises a blend of the first biodegradable polymer and the second biodegradable polymer, the content of the first biodegradable polymer contained in the sheath part based on the total weight of the blend contained in the sheath part may be greater than the content of the first biodegradable polymer contained in the intermediate part based on the total weight of the blend contained in the intermediate part, and the content of the first biodegradable polymer contained in the intermediate part based on the total weight of the blend contained in the intermediate part may be greater than the content of the first biodegradable polymer contained in the core part based on the total weight of the blend contained in the core part.

In another embodiment, the core part, the intermediate part, and the sheath part each may comprise the first biodegradable polymer, the second biodegradable polymer, or a blend thereof. The weight ratio of the first biodegradable polymer to the second biodegradable polymer contained in each of the core part, the intermediate part, and the sheath part may be the highest in the sheath part and the lowest in the core part. In addition, the weight ratio of the second biodegradable polymer to the first biodegradable polymer contained in each of the core part, the intermediate part, and the sheath part may be the highest in the core part and the lowest in the sheath part.

In another embodiment, the implant may comprise at least two intermediate parts interposed between the core part and the sheath part, each of the intermediate parts comprises a blend of the first biodegradable polymer and the second biodegradable polymer, and the content of the first biodegradable polymer contained in each of the intermediate parts based on the total weight of the blend contained in each of the intermediate parts may be higher as the intermediate part is closer to the sheath part. In addition, the content of the second biodegradable polymer contained in each of the intermediate parts based on the total weight of the blend contained in each of the intermediate parts may be higher as the intermediate part is closer to the core part.

For example, the sheath part is composed of the first biodegradable polymer (e.g., PHA), and the mixing ratio of the second biodegradable polymer (e.g., PLA) may gradually increase toward the core part. The difference in the content of the first biodegradable polymer or the second biodegradable polymer that increases or decreases for each intermediate part may be, for example, 5% by weight to 50% by weight.

The size and thickness of the core part, the intermediate part, and the sheath part may vary depending on the content ratio of the first biodegradable polymer and the second biodegradable polymer contained in the implant.

The shape of the implant is not particularly limited. For example, it may be cylindrical (or pin-shaped), square pillar-shaped, or plate-shaped.

The implant may have a porous structure.

For example, the implant may comprise a plurality of pores.

Specifically, the implant may have a porous structure comprising pores with a size of 10 nm to 10 µm.

In addition, the implant may have a roughness or a pattern on its surface.

In an embodiment, the implant may have a certain roughness on its surface.

In an embodiment, the implant may have a surface roughness with a depth of 1 µm to 10 µm. More specifically, the implant may have micro-concave-convex parts on the surface with a depth of 1 µm to 10 µm and a spacing of 1 µm to 10 µm.

In an embodiment, the implant may further have a small roughness within a large roughness on the surface. More specifically, the implant may have first micro-concave-convex parts on its surface with a depth of 1 µm to 10 µm and a spacing of 1 µm to 10 µm, and the first micro-concave-convex part may have second micro-concave-convex parts with a depth of 0.1 µm to 1 µm and a spacing of 0.1 µm to 1 µm on the surface (see Fig. 2).

In another embodiment, the implant may have a certain pattern on its surface.

Specifically, the implant may have a grid pattern, a wave pattern, or a stripe pattern on the surface with a spacing of 1 µm to 10 µm.

As the implant according to the present disclosure comprises a first biodegradable polymer comprising a copolymeric polyhydroxyalkanoate (PHA) and a second biodegradable polymer, it exhibits appropriate strength and stability for orthopedic implants, for example, a material for hard tissues fixation such as pins, screws, and fixation plates, and has excellent osteogenic and osseointegration properties.

For example, the implant may have a tensile strength of 35 MPa or more, 40 MPa or more, 45 MPa or more, or 50 MPa or more, and 75 MPa or less, 70 MPa or less, 65 MPa or less, or 60 MPa or less. As a specific example, the tensile strength of the implant may be 40 MPa to 70 MPa. More specifically, the tensile strength of the implant may be 40 MPa to 60 MPa or 50 MPa to 60 MPa.

The implant may have an elongation of 2% or more, 3% or more, 3.2% or more, or 4% or more, and 10% or less, 9% or less, 8% or less, 7% or less, 6% or less, or 5% or less. As a specific example, the elongation of the implant may be 3.2% to 10%. More specifically, the elongation of the implant may be 3.2% to 7%.

The tensile strength and elongation of the implant may be measured by, for example, preparing a specimen with a diameter of 1.5 mm and a length of 50 mm.

The implant, in a cytotoxicity test using an eluate, may exhibit a cell viability of 70% or more relative to the cell viability of 100% of an elution solvent control group. In addition, the implant, in a cytotoxicity test using an eluate, may exhibit a cell viability of 75% or more, 80% or more, or 81% or more, relative to the cell viability of 100% of an elution solvent control group. In addition, the implant, in a cytotoxicity test using an eluate, may exhibit a cell viability of 70% to 90%, 80% to 90%, or 81% to 90%, relative to the cell viability of 100% of an elution solvent control group. Specifically, in the cytotoxicity test, the implant is eluted with an elution solvent (e.g., cell culture medium), cells are treated with the eluate, and the viability of the cells is measured in 48 hours. A relative value to the cell viability of 100% for a control group treated with only the elution solvent is then calculated.

In addition, when the implant is implanted into a fractured femur tissue of a rat, the stiffness of the femur tissue harvested after 2 months from the implantation may be 120 N/mm or more. Specifically, when the implant is implanted into a fractured femur tissue of a rat, the stiffness of the femur tissue harvested after 2 months from the implantation may be 130 N/mm or more. For example, when the implant is implanted into a fractured femur tissue of a rat, the stiffness of the femur tissue harvested after 2 months from the implantation may be 120 N/mm to 500 N/mm, 130 N/mm to 500 N/mm, 130 N/mm to 400 N/mm, or 150 N/mm to 300 N/mm.

In addition, when the implant is implanted into a fractured femur tissue of a rat, the stiffness of the femur tissue harvested after 4 months from the implantation may be 170 N/mm or more. For example, when the implant is implanted into a fractured femur tissue of a rat, the stiffness of the femur tissue harvested after 4 months from the implantation may be 170 N/mm to 700 N/mm, 200 N/mm to 600 N/mm, or 250 N/mm to 500 N/mm.

The stiffness of a femur tissue may be measured by, for example, a three-point bending test using a biomechanical tester. The test may be carried out by applying the standards (ASTM F1161 and ASTM F382) generally used for testing ceramic or fractured composite plates. In an embodiment, a specimen is placed on a test jig with the support span spacing set to 16 mm such that the fracture site is located on the upper jig, and a load of 3 kN is applied at a rate of 5 mm/minute. The stiffness may be measured by performing the test until the specimen is broken.

### Process for manufacturing the implant

The process for manufacturing an implant according to the present disclosure comprises preparing a biodegradable polymer comprising a first biodegradable polymer and a second biodegradable polymer; and forming an implant using the biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

In an embodiment, the forming may be injection molding.

The injection molding may be carried out by, for example, extrusion using a twin-screw extruder or a single-screw extruder, followed by injection using an injection machine, and then performing subsequent processing such as annealing. The mold used for the injection molding may be, for example, a cold runner mold, a hot runner mold, or a pinpoint mold.

The temperature during the extrusion may be, for example, 100°C to 210°C, the main screw rpm range may be, for example, 100 rpm to 250 rpm, and the feeder rpm range may be, for example, 4 rpm to 40 rpm.

The temperature during the injection may be, for example, 150°C to 200°C, the injection pressure may be 1,000 bar to 1,800 bar, the injection speed may be, for example, 0.5 mm/s to 100 mm/s, and the holding pressure range may be, for example, 300 bar to 1,200 bar, and the holding pressure time may be, for example, 1 s to 10 s.

The temperature during the annealing may be, for example, 80°C to 110°C, and the annealing time may be, for example, 30 minutes to 2 hours.

In another embodiment, the forming may be 3D printing molding.

The 3D printing molding may be carried out under the conditions of a printing temperature of, for example, 150°C to 200°C, an injection speed of, for example, 200 mm/minute to 400 mm/minute, a pressure of, for example, 200 kPa to 400 kPa, and a printing duration time of, for example, 60 minutes or less.

In another embodiment, the surface of the implant may be modified to enhance cell adhesion and strengthen fixation within the tissue.

In an embodiment, the process may further comprise, after the forming, imparting a roughness or a pattern to the surface through blasting, molding, or laser treatment.

In another embodiment, the process may further comprise, after the forming, imparting hydrophilicity to the surface through plasma treatment.

In another embodiment, the process may further comprise, after the forming, imparting a roughness or a pattern to the surface through blasting, molding, or laser treatment; and imparting hydrophilicity to the surface through plasma treatment.

### Composition for the implant

The composition for an implant according to the present disclosure comprises a first biodegradable polymer and a second biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

In an embodiment, the composition for an implant may comprise a blend of the first biodegradable polymer and the second biodegradable polymer.

In another embodiment, the copolymeric polyhydroxyalkanoate (PHA) may further comprise a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

In another embodiment, the first biodegradable polymer may further comprise at least one polymer selected from the group consisting of poly(3-hydroxybutyrate) (P3HB), poly(4-hydroxybutyrate) (P4HB), poly(3-hydroxyhexanoate) (P3HH), poly(3-hydroxybutyrate-co-3-hydroxyhexanoate) (P3HB-3HH), poly(3-hydroxyoctanoate) (P3HO), and poly(3-hydroxybutyrate-co-3-hydroxyoctanoate) (P3HB-3HO).

In another embodiment, the second biodegradable polymer is one, or a mixture of two or more, selected from synthetic biodegradable polymers and natural biodegradable polymers excluding polyhydroxyalkanoate (PHA), the synthetic biodegradable polymer may comprise polylactic acid (PLA), polycaprolactone (PCL), polyglycolic acid (PGA), poly(lactic acid-co-caprolactone) (PLCL), poly(lactic acid-co-glycolic acid) (PLGA), polybutylene succinate (PBS), and poly(butylene adipate-co-terephthalate) (PBAT); and the natural biodegradable polymer may comprise starch, silk fibroin, chitosan, chitin, cellulose, collagen, and gelatin.

In another embodiment, the copolymeric polyhydroxyalkanoate (PHA) may be included in an amount of 10% by weight to 50% by weight based on the total weight of the first biodegradable polymer and the second biodegradable polymer.

In another embodiment, the composition for an implant may further comprise at least one additive selected from bioactive glass fiber or ceramic.

In another embodiment, the composition for an implant may further comprise at least one drug for enhancing bone regeneration.

Specific details of the first biodegradable polymer, the second biodegradable polymer, their mixing ratio, the content of a repeat unit derived from 4-hydroxybutyrate (4-HB) in the copolymeric polyhydroxyalkanoate (PHA), and the type and content of additives and drugs are exemplified in the description of the implant above.

The composition for an implant can be used in manufacturing an implant according to an embodiment of the present disclosure. For example, it can be used in manufacturing an implant according to the process described above.

### Mode for the Invention

Hereinafter, the present disclosure will be described in more detail with reference to the following examples. But the following Examples are intended to illustrate the present disclosure, and the scope of the Examples is not limited thereto only.

### Examples 1 to 3 and Comparative Example 1: Manufacture of an implant

Materials and equipments are as follows.
- PLA: PLLA (BBCA)
- PHA: P(3HB-4HB) (4-HB 10%, CJ CheilJedang)
- Extruders (OMEGA 20 from STEER, BA 19 from Bautech)
- Injection molding machine (E-victory 170/80 from Engel)

PLLA and P(3HB-4HB) were compounded at different weight ratios as shown in the table below.

**[Table 1]**

| | Example 1 | Example 2 | Example 3 | Comparative Example 1 |
|---|---|---|---|---|
| P(3HB-4HB) | 30 | 20 | 10 | - |
| PLLA | 70 | 80 | 90 | 100 |
| Total content | 100 | 100 | 100 | 100 |

The following two types of twin-screw extruders were used to carry out compounding.

BA 19 - Temperature range: 100-170°C, main screw rpm: 150, feeder rpm: 7-8, torque range: 36-42

Omega 20 - Temperature range: 120-195°C, main screw rpm: 250, feeder rpm: 25-35, torque range: 39-46

Drying was carried out at 60°C overnight and then at 80°C for 6 hours.

Injection conditions are as follows. Temperature: 160-180°C, mold temperature: 30-70°C, pressure: 1,100-1,500 bar, injection speed: 5-120 mm/s, holding pressure: 300-600 bar, holding pressure time: 3 s, metering amount: 12-15 mm, metering speed: 40 rpm, back pressure: 40 bar, holding pressure switching time: 10 s, cooling time: 15-30 s.

As a result, pin-shaped implants with a diameter of 1.5 mm and a length of 50 mm were manufactured with various compositions.

### Test Example 1: Strength and elongation

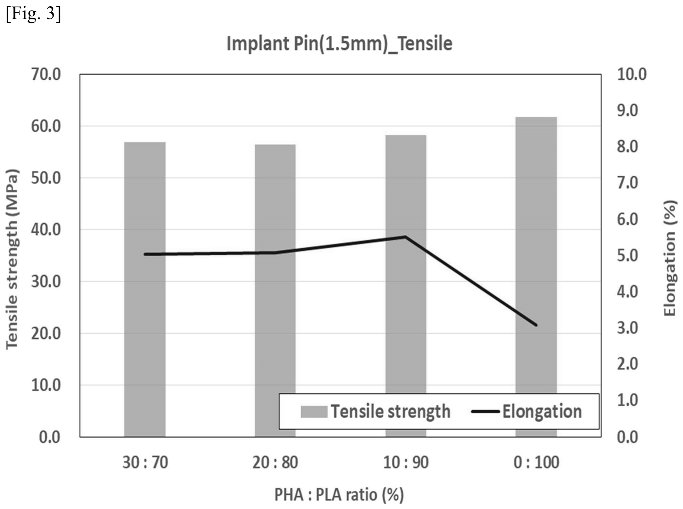

The equipment used was UTM (34SC-1 from Instron), and an implant specimen in the form of a pin with a diameter of 1.5 mm and a length of 50 mm was prepared. The test conditions were a load cell load of 1 kN, a jig distance of 20 mm, and a tensile speed of 20 mm/minute. The test results are shown in the table below and Fig. 3.

**[Table 2]**

| | Unit | Example 1 | Example 2 | Example 3 | Comparative Example 1 |
|---|---|---|---|---|---|
| Tensile strength | MPa | 56.9 | 56.4 | 58.2 | 61.7 |
| Elongation | % | 5.0 | 5.1 | 5.5 | 3.1 |

As can be seen from the test results, in Examples 1 to 3 in which 10 to 30% by weight of P(3HB-4HB) was included, the elongation was increased, thereby imparting flexibility to the material, with little difference in tensile strength, as compared with Comparative Example 1 in which P(3HB-4HB) was not included. Thus, they are suitable as an implant material.

In addition, tests were conducted using P(3HB-4HB) (CJ CheilJedang) in which the content of 4HB was varied to 6%, 8%, and 10%. Implants were manufactured in the same manner as in Example 1 using P(3HB-4HB) with various copolymer compositions and PLLA in a weight ratio of 3:7. The tensile strength and elongation of the implants thus manufactured were tested as before, and the results are shown in the table below.

**[Table 3]**

| P(3HB-4HB):PLLA = 3:7 | | | | |
|---|---|---|---|---|
| Content of 4HB in P(3HB-4HB) | (%) | 6 | 8 | 10 |
| Tensile strength | (MPa) | 61.46 | 56.88 | 56.52 |
| Elongation | (%) | 6.31 | 6.27 | 6.11 |

### Test Example 2: Cytotoxicity (in vitro)

### (1) Test method

The cytotoxicity evaluation was conducted based on the international standard ISO 10993-1:12 and the "Considerations for applying GLP to medical device biological safety testing (cytotoxicity testing guidelines)" published by the Ministry of Food and Drug Safety. Positive control materials, negative control materials, elution conditions, cell lines, and the like were used based on the guidelines, as follows.
- Positive control group: polyurethane stabilized with organotin
- Negative control group: high-density polyethylene (standard material for cytotoxicity evaluation)
- Elution solvent: cell culture medium (RPMI-1640 + 10% fetal bovine serum + 1% antibiotics)
- Elution conditions: 0.2 g/mL, 37±2°C, 24±2 hours
- Cell line: WI-38 (human lung fibroblast cell line)

Cytotoxicity evaluation is divided into direct and indirect methods. The direct method is a test that confirms changes in cells by bringing a test sample into direct contact with cells. The indirect method is a test in which a test sample is eluted in a cell culture medium, cells are treated with the eluate, and changes in the cells in 48 hours are observed.

First, cells were distributed in a 24-well plate and left in an incubator for 24 hours. It was then checked whether the cells had proliferated up to 80%. Thereafter, for the direct method, the test sample was injected into the cell culture plate. For the indirect method, the cell culture medium was replaced with the eluate. Upon incubation for 48 hours, changes in the cells were observed.

After 48 hours, live/dead cell fluorescence staining was used for the direct method. For the indirect method, qualitative analysis was performed through observation of cell shape, followed by quantitative analysis using the WST-1 assay. The evaluation during the analysis was conducted according to the guidelines.

### (2) Test results

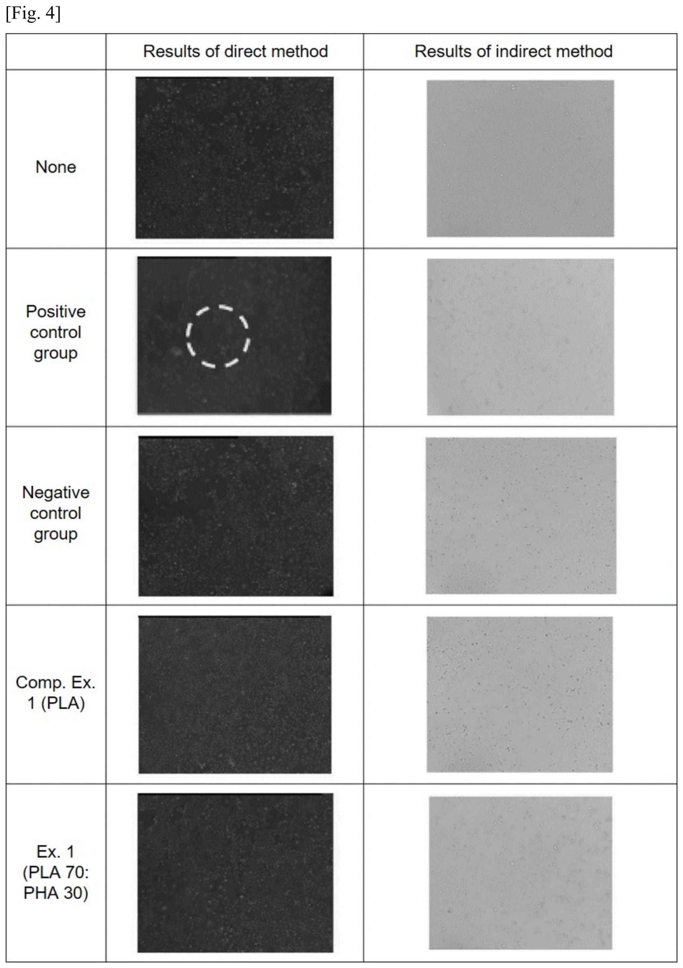

The results of live/dead cell fluorescence staining according to the direct method are shown in the table below and in Fig. 4. As a result of the test, both Comparative Example 1 and Example 1 containing biodegradable polymers had fewer dead cells than the positive control group and were similar to the negative control group.

**[Table 4]**

| | None | Positive control group | Negative control group | Comparative Example 1 (PLA) | Example 1 (PLA 7:PHA 3) |
|---|---|---|---|---|---|
| Result | No changes | Dead cells observed | No changes | No changes | No changes |

In addition, the cell shape was observed after the test according to the indirect method and is shown in Fig. 4. As a result of the test, in Example 1, it was observed that the cells maintained the fibroblast shape, as adhered, and proliferated by 80% or more. In addition, in Comparative Example 1, it was confirmed that the cells proliferated a lot and filled the culture plate similar to the negative control group.

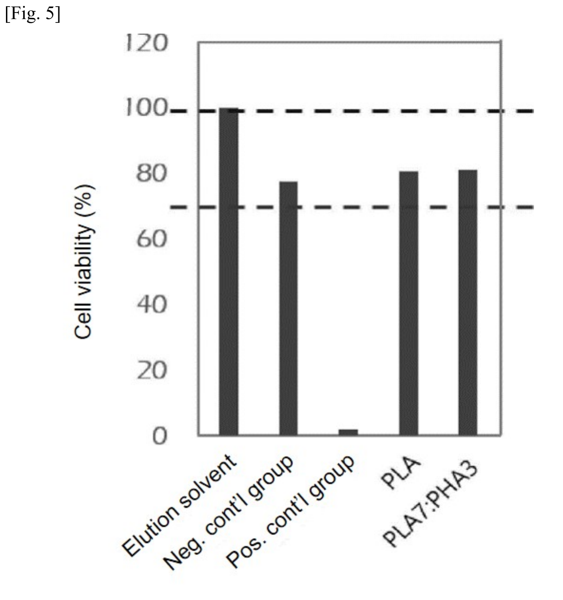

In addition, the cell viability was measured (WST-1 assay) after the test according to the indirect method and is shown in Fig. 5. As a result of the test, when the cell viability of the elution solvent control group was 100%, Comparative Example 1 (PLA 100%) showed a viability of 80.9%, and Example 1 (PLA:PHA = 7:3) showed a viability of 81.1%. If the cell viability is reduced by 30% or more, it is considered cytotoxic based on the Ministry of Food and Drug Safety guidelines. It was confirmed that both Comparative Example 1 and Example 1 showed no cytotoxicity.

### Test Example 3: Osteogenic differentiation induction (in vitro)

### (1) Test method

The osteogenic differentiation induction test is conducted to check the degree of osteogenic differentiation when a test sample is into direct contact with bone cells. It was conducted using the cell line SaOS-2 (human osteosarcoma cell line). The cell culture medium (using osteogenesis promotion medium) was changed once every three days and cultured for two weeks. Alizarin Red stain was performed to check whether calcium was produced to evaluate the degree of osteogenic differentiation.

### (2) Test results

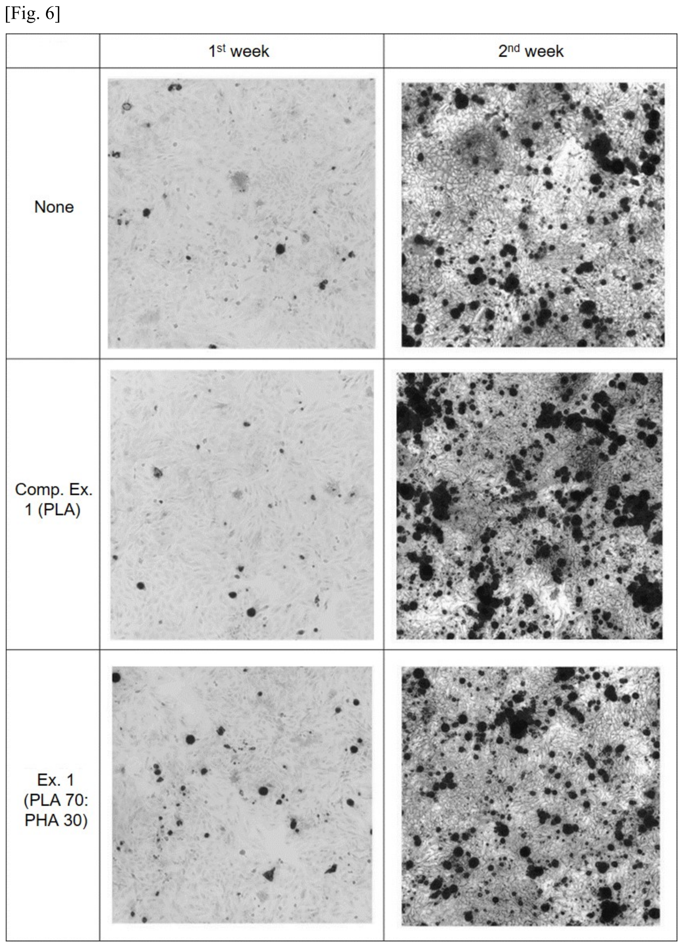

The results of the osteogenic differentiation induction test are shown in Fig. 6. As a result of the test, in the first week, Example 1 (PLA/PHA = 7:3) showed slightly more calcium deposition staining than the other groups, indicating that osteogenic differentiation occured slightly faster than the other groups. However, in the second week, it was confirmed that osteogenic differentiation was induced in all groups without differences between groups.

### Test Example 4: Rat femur fracture (in vivo)

### (1) Test method

A PHAs-based orthopedic pin-type implant was used to carry out an *in vivo* test through a rat fracture model. Based on existing literature and previous research, Sprague Dawley's femur was osteotomized and an implant was inserted. Femur samples in Comparative Example 1 (PLA 100%) and Example 1 (PLA/PHA = 7:3) were collected after 2 and 4 months from the implantation to perform histological, radiological, and biomechanical analyses. The results are compared.

The procedure was performed as a femur fracture model for 12-week-old rats. As shown in the table below, out of a total of 25 rats, 12 rats were implanted with a PLA implant, and the remaining 13 rats were implanted with an implant of Example 1 (PLA/PHA = 7:3), and observed for 4 months. In the 2^{nd} month, 3 rats implanted with the PLA implant and 3 rats implanted with the implant of Example 1 (PLA/PHA = 7:3) were sacrificed to produce tissue slides, and 3 rats implanted with the implant of Comparative Example 1 (PLA 100%) and 4 rats implanted with the implant of Example 1 (PLA/PHA = 7:3) were sacrificed for micro-CT imaging and evaluation of femoral flexion strength. Femur tissues of a total of 13 rats were collected. In the 4^{th} month, 3 rats implanted with the PLA implant and 3 rats implanted with the implant of Example 1 (PLA/PHA = 7:3) were sacrificed to produce tissue slides, and 3 rats implanted with the PLA implant and 3 rats implanted with the implant of Example 1 (PLA/PHA = 7:3) were sacrificed for micro-CT imaging and evaluation of femoral flexion strength. Femur tissues of a total of 12 rats were collected. Thereafter, histopathological, radiological, and biomechanical evaluations were carried out through tissue staining, micro-CT imaging, and flexural strength evaluation.

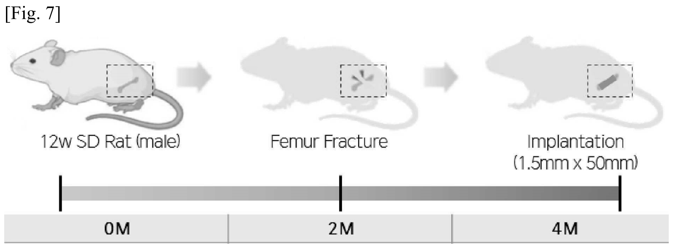

The model for the rat femur fracture test is explained in Fig. 7 and summarized in the table below.

**[Table 5]**

| Month 0 | 2^{nd} Month | 4^{th} Month |
|---|---|---|
| Implant of Comparative Example 1 (PLA 100%) was implanted in 12 rats | 6 rats sacrificed | 6 rats sacrificed |
| | 3 rats for histopathological analysis; 3 rats for radiological and biomechanical analyses | 3 rats for histopathological analysis; 3 rats for radiological and biomechanical analyses |
| Implant of Example 1 (PLA/PHA = 7:3) was implanted in 13 rats | 7 rats sacrificed | 6 rats sacrificed |
| | 3 rats for histopathological analysis; 4 rats for radiological and biomechanical analyses | 3 rats for histopathological analysis; 3 rats for radiological and biomechanical analyses |

For the histopathological analysis, femur samples were stained with H&E stain, and basic bone morphology, changes in the fracture site, implant bone contact surface, and new bone formation were observed through microscopic findings. For the radiological analysis, the degree of bone union of the femur was evaluated based on micro-CT images. For the biomechanical analysis, a femur sample was fixed to a biomechanical test machine to carry out a three-point bending experiment.

During the procedure for the pin-type implant of Comparative Example 1 (PLA 100%), a very serious problem occurred in which the pin broke while fixing it to the bone; thus, it was determined to be difficult to use as a surgical implant. In Example 1 (PLA/PHA = 7:3), there were no problems that occurred in Comparative Example 1 (PLA 100%); thus, it was confirmed that Example 1 (PLA/PHA = 7:3) was more suitable as a surgical implant. That is, it was confirmed during the surgical procedure that the pin-type implant of Example 1 (PLA/PHA = 7:3) had improved physical properties and was more suitable as a surgical implant than that of Comparative Example 1 (PLA 100%).

None of the 25 rats used in the experiment showed any visible signs of inflammation at the wound site. All survived without any particular problems for 2 and 4 months. Femur samples from 25 rats were successfully collected in the 2^{nd} and 4^{th} months after surgery.

### (2) Histopathological evaluation

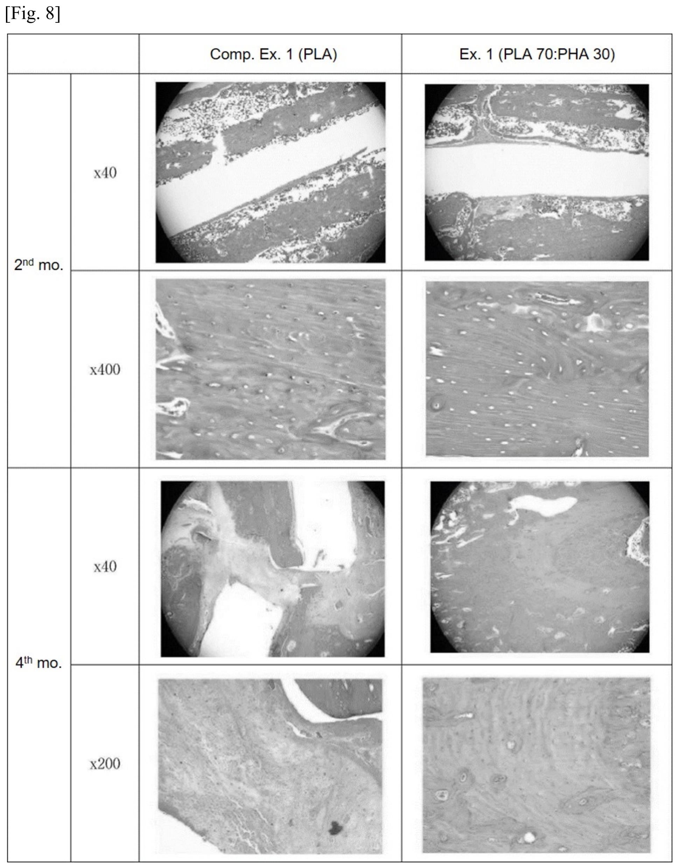

Histopathological analysis of the collected femur samples was conducted with the Department of Pathology at Konyang University. A slide was prepared using a paraffin block and a microtome centered on the osteotomy site of the femur, and H&E staining was performed to observe the arrangement of the bone matrix and the nuclei and cytoplasm of osteocytes to compare and analyze the progress of bone union in the control group and test group. In addition, the pin insertion site and contact surface of the femur were checked to determine whether there was a foreign body reaction. The results are shown in Fig. 8.

As a result of H&E staining of the femur sample collected in the 2^{nd} month, the bone matrix of Example 1 (PLA/PHA = 7:3) was more regularly and well organized than that of Comparative Example 1 (PLA 100%), and the cytoplasm of bone cells was small and had a spindle shape, indicating that Example 1 showed progress in mature bone formation as compared with Comparative Example 1.

In addition, the femur sample collected in the 4^{th} month showed that the bone matrix of Example 1 (PLA/PHA = 7:3) was more regularly and well organized than that of Comparative Example 1 (PLA 100%), and the cytoplasm of bone cells was small and had a uniform spindle shape, indicating that Example 1 showed progress in mature bone formation as compared with Comparative Example 1.

### (3) Radiological analysis

Radiological analysis of the collected femur samples was conducted with the analysis team of the Osong Medical Innovation Foundation (KBio Health). The radiological analysis was conducted using micro-CT. The bone union of the osteotomy surface was observed using 2D and 3D images, and the gap, bone bridge, and hypertrophy patterns of the fracture surface were analyzed. In addition, the callus volume as a quantitative parameter of bone union and the bone marrow density (BMD) as a qualitative parameter thereof in the ROI (range of interest) area of the osteotomy site were measured using micro-CT to analyze the degree of bone union.

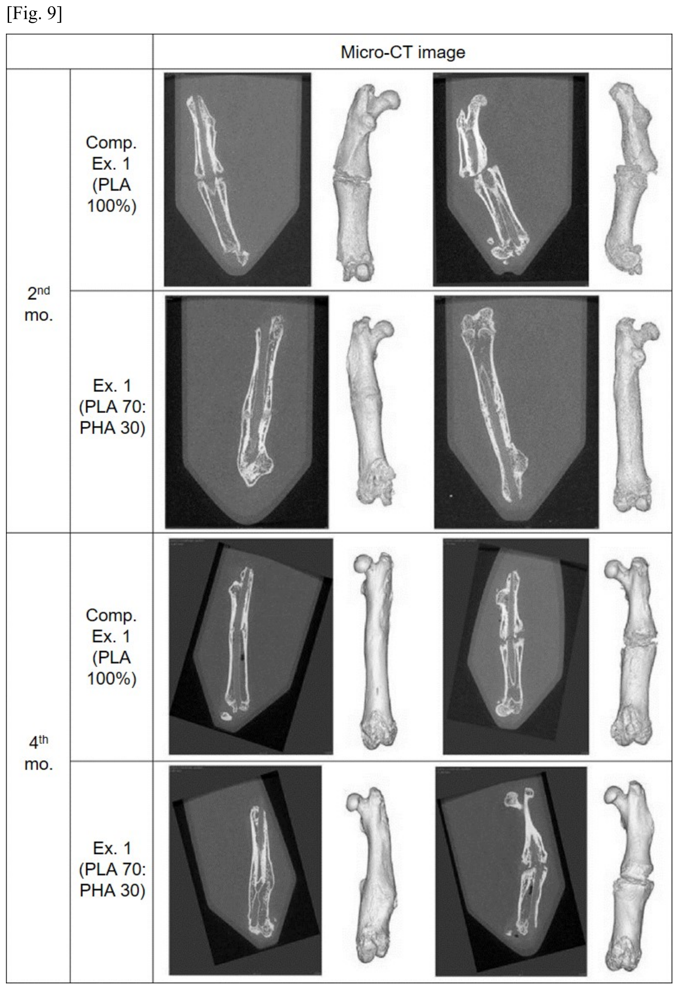

Fig. 9 shows micro-CT 2D and 3D images of the samples in the 2^{nd} and 4^{th} months. Fig. 9(a) shows images of a sample in which the implant of Comparative Example 1 (PLA 100%) was implanted, and Fig. 9(b) shows images of a sample in which the implant of Example 1 (PLA/PHA = 7:3) was implanted.

As a result of the test in the 2^{nd} month, a gap in the bone fracture surface was observed in all three samples of Comparative Example 1 (PLA 100%), and no bone bridge was observed. In particular, one sample of Comparative Example 1showed displacement of the fracture surface and pins, indicating that refracture had occurred. In contrast, in Example 1 (PLA/PHA = 7:3), a gap in the fracture surface was observed in two out of the four samples, and bone bridges were observed in two samples. In conclusion, bone union was not achieved in all three samples in Comparative Example 1; and in Example 1, bone union was achieved in two out of four samples (50%).

As a result of the test in the 4^{th} month, a gap in the bone surface was observed in two out of three samples of Comparative Example 1, and a bone bridge was observed in one sample. In Example 1, a gap in the fracture surface was observed in one out of three samples, and a bridge was observed in two samples. In conclusion, in Comparative Example 1, bone union was achieved in one out of three samples; and in Example 1, bone union was achieved in two out of four samples.

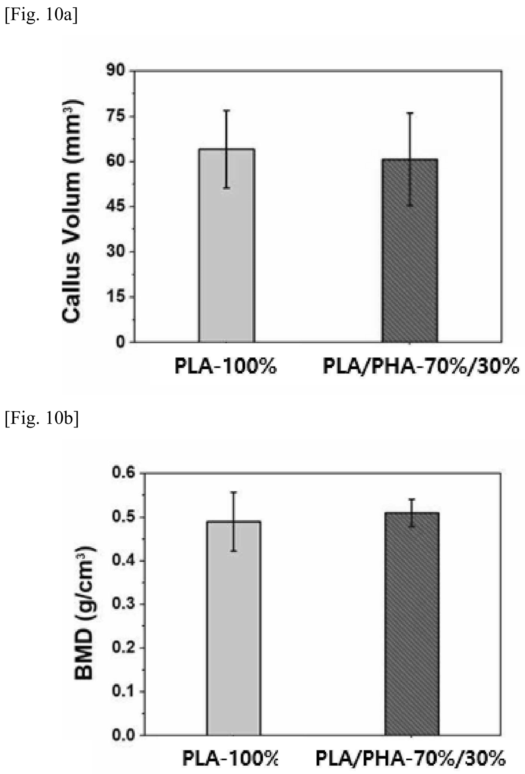

The results of radiological analysis of the 2^{nd}-month sample are shown in the table below and Figs. 10a and 10b. As a result of analysis of the 2^{nd}-month sample, there was no significant difference between Example 1 and Comparative Example 1 in callus volume and BMD.

**[Table 6]**

| Comparative Example 1 (PLA 100%) | Bone volume (mm²) | Tissue volume (mm³) | Bone volume/tissue volume (%) | BMD (g/cm³) |
|---|---|---|---|---|
| #1 | 71.71 | 529.20 | 13.55 | 0.4005 |
| #2 | 74.58 | 529.20 | 14.09 | 0.5420 |
| #3 | 46.21 | 529.20 | 8.73 | 0.5420 |
| Avg. | 64.17 | 529.20 | 0.12 | 0.4900 |
| Std. deviation | 12.75 | 0.00 | 0.02 | 0.0667 |

**[Table 7]**

| Example 1 (PLA/PHA = 7:3) | Bone volume (mm²) | Tissue volume (mm³) | Bone volume/tissue volume (%) | BMD (g/cm³) |
|---|---|---|---|---|
| #1 | 76.59 | 529.20 | 14.47 | 0.5475 |
| #2 | 63.31 | 529.20 | 11.96 | 0.5190 |
| #3 | 35.43 | 529.20 | 6.69 | 0.4619 |
| #4 | 47.83 | 529.20 | 12.82 | 0.5103 |
| Avg. | 60.79 | 529.20 | 0.11 | 0.5097 |
| Std. deviation | 15.40 | 0.00 | 0.03 | 0.0308 |

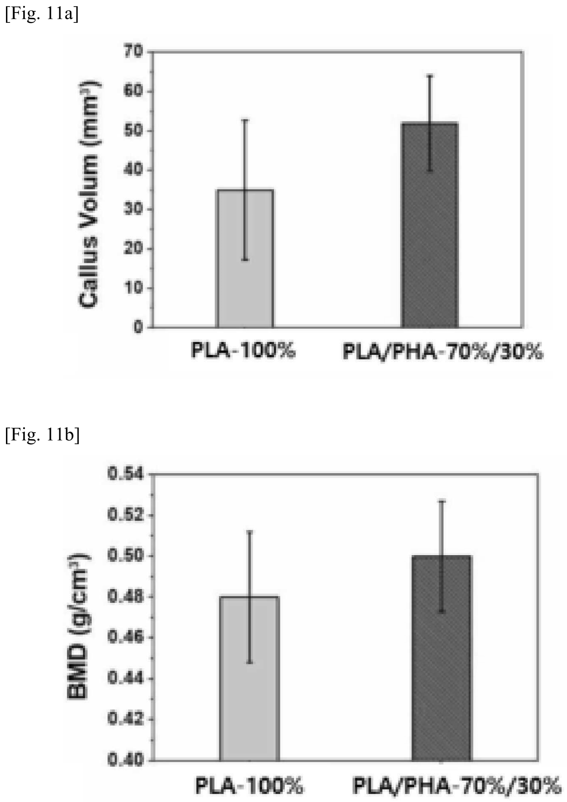

The results of radiological analysis of the 4^{th}-month sample are shown in the table below and Figs. 11a and 11b. As a result of analysis of the 4^{th}-month sample, Example 1 was slightly increased as compared with Comparative Example 1 in callus volume and BMD.

**[Table 8]**

| Comparative Example 1 (PLA 100%) | Bone volume (mm²) | Tissue volume (mm³) | Bone volume/tissue volume (%) | BMD (g/cm³) |
|---|---|---|---|---|
| #1 | 22.487 | 529.20 | 4.2 | 0.437 |
| #2 | 60.059 | 529.20 | 11.3 | 0.513 |
| #3 | 22.487 | 529.20 | 4.2 | 0.490 |
| Avg. | 35.011 | 529.20 | 6.62 | 0.480 |
| Std. deviation | 17.712 | 0.00 | 0.03 | 0.032 |

**[Table 9]**

| Example 1 (PLA/PHA = 7:3) | Bone volume (mm²) | Tissue volume (mm³) | Bone volume/tissue volume (%) | BMD (g/cm³) |
|---|---|---|---|---|
| #1 | 41.184 | 529.20 | 7.8 | 0.476 |
| #2 | 68.929 | 529.20 | 13.0 | 0.537 |
| #3 | 45.963 | 529.20 | 8.7 | 0.486 |
| Avg. | 52.025 | 529.20 | 9.83 | 0.500 |
| Std. deviation | 12.111 | 0.00 | 0.02 | 0.027 |

### (4) Biomechanical analysis

A three-point bending test was carried out for femur samples collected using a biomechanical test machine. The stiffness (N/mm), maximum load (N), and maximum deformation (mm) of the samples were measured to evaluate biomechanical properties.

The three-point bending biomechanical test was performed referring to the standards commonly used for testing ceramic or fracture composite plates (ASTM F1161 Standard Test Method for Flexural Strength of Advanced Ceramics at Ambient Temperature, ASTM F382 Standard Specification and Test Method for Metallic Bone Plates).

Specific test conditions and test methods are as follows.
- support span: 16 mm
- loading rate: 5 mm/minute
- test environment: dry at ambient, room temperature
- test equipment: MTS Acumen Test system (MTS system Corp. Mn, USA)
- load capacity: 3 kN
- A specimen was placed on a test jig with the support span spacing set to 16 mm such that the fracture site was located on the upper jig. A load of 3 kN was applied to the specimen at a rate of 5 mm/minute. The load and deformation were measured by performing the test until the specimen was broken.

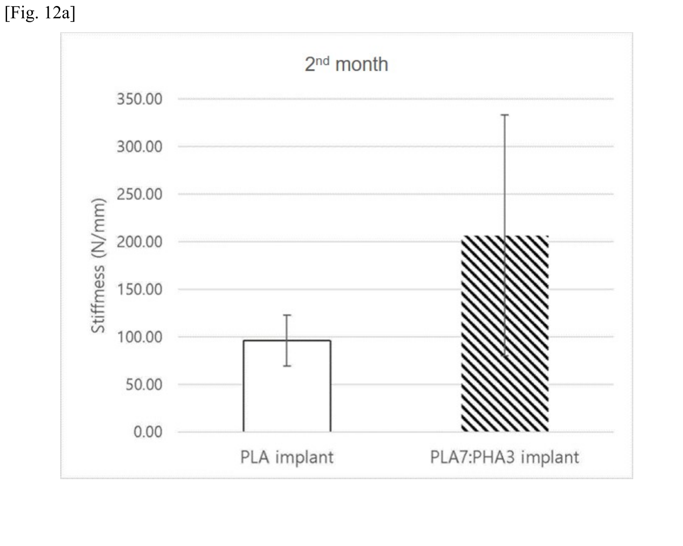

The results of biomechanical analysis of the 2^{nd}-month sample are shown in the tables below. In addition, the measurement results of stiffness of the 2^{nd}-month sample are shown in Fig. 12a.

**[Table 10]**

| Comparative Example 1 (PLA 100%) | Stiffness (N/mm) | Max. load (N) | Max. deformation (mm) |
|---|---|---|---|
| #1 | 117.39 | 79.08 | 1.42 |
| #2 | 112.36 | 91.82 | 1.31 |
| #3 | 58.26 | 54.07 | 1.91 |
| Avg. | 96.00 | 74.99 | 1.55 |
| Std. deviation | 26.77 | 15.68 | 0.26 |

**[Table 11]**

| Example 1 (PLA/PHA = 7:3) | Stiffness (N/mm) | Max. load (N) | Max. deformation (mm) |
|---|---|---|---|
| #1 | 132.71 | 88.01 | 0.72 |
| #2 | 130.19 | 103.48 | 1.10 |
| #3 | 425.79 | 117.05 | 0.57 |
| #4 | 137.96 | 95.83 | 1.05 |
| Avg. | 206.66 | 101.09 | 0.86 |
| Std. deviation | 126.54 | 10.71 | 0.22 |

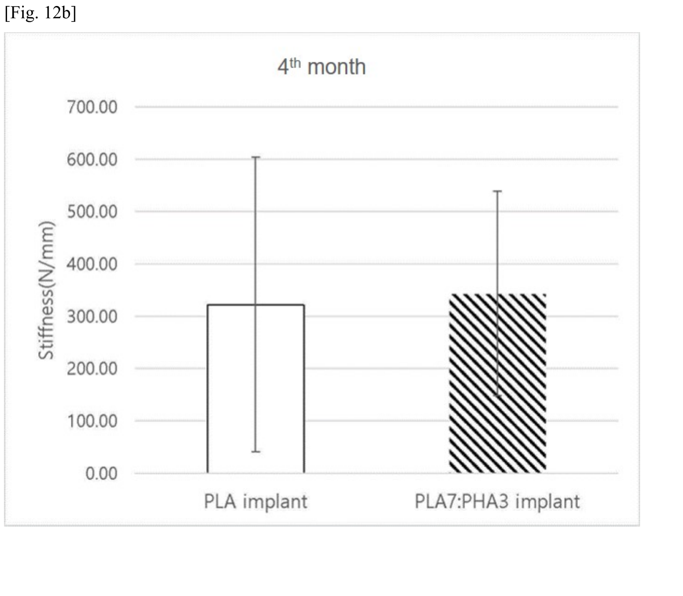

The results of biomechanical analysis of the 4^{th}-month sample are shown in the tables below. In addition, the measurement results of stiffness of the 4^{th}-2ndmonth sample are shown in Fig. 12b.

**[Table 12]**

| Comparative Example 1 (PLA 100%) | Stiffness (N/mm) | Max. load (N) | Max. deformation (mm) |
|---|---|---|---|
| #1 | 715.05 | 144.88 | 0.30 |
| #2 | 68.94 | 88.66 | 2.07 |
| #3 | 183.09 | 135.83 | 0.86 |
| Avg. | 322.36 | 123.12 | 1.08 |
| Std. deviation | 281.56 | 24.65 | 0.74 |

**[Table 13]**

| Example 1 (PLA/PHA = 7:3) | Stiffness (N/mm) | Max. load (N) | Max. deformation (mm) |
|---|---|---|---|
| #1 | 617.49 | 211.21 | 0.64 |
| #2 | 176.79 | 142.46 | 1.36 |
| #3 | 236.02 | 172.86 | 1.07 |
| Avg. | 343.43 | 175.51 | 1.02 |
| Std. deviation | 195.29 | 28.13 | 0.30 |

As a result of biomechanical analysis, the biomechanical strength of Example 1 (PLA/PHA = 7:3) was measured to be superior to that of Comparative Example 1 (PLA 100%) in the 2^{nd} and 4^{th} months.

## Claims

1. An implant, which comprises a first biodegradable polymer and a second biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

2. The implant of claim 1, wherein the implant comprises a blend of the first biodegradable polymer and the second biodegradable polymer.

3. The implant of claim 1, wherein the implant comprises a core part and a sheath part, the sheath part comprises the first biodegradable polymer, and the core part comprises the second biodegradable polymer.

4. The implant of claim 1, wherein the implant comprises a core part and a sheath part,
the core part and the sheath part each comprise a blend of the first biodegradable polymer and the second biodegradable polymer, and
the content of the first biodegradable polymer contained in the sheath part based on the total weight of the blend contained in the sheath part is greater than the content of the first biodegradable polymer contained in the core part based on the total weight of the blend contained in the core part.

5. The implant of claim 4, wherein the implant comprises at least one intermediate part disposed between the core part and the sheath part,
at least one intermediate part comprises a blend of the first biodegradable polymer and the second biodegradable polymer,
the content of the first biodegradable polymer contained in the sheath part based on the total weight of the blend contained in the sheath part is greater than the content of the first biodegradable polymer contained in the intermediate part based on the total weight of the blend contained in the intermediate part, and
the content of the first biodegradable polymer contained in the intermediate part based on the total weight of the blend contained in the intermediate part is greater than the content of the first biodegradable polymer contained in the core part based on the total weight of the blend contained in the core part.

6. The implant of claim 1, wherein the implant has a porous structure comprising pores with a size of 10 nm to 10 µm.

7. The implant of claim 1, wherein the copolymeric polyhydroxyalkanoate (PHA) further comprises a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

8. The implant of claim 1, wherein the first biodegradable polymer further comprises at least one polymer selected from the group consisting of poly(3-hydroxybutyrate) (P3HB), poly(4-hydroxybutyrate) (P4HB), poly(3-hydroxyhexanoate) (P3HH), poly(3-hydroxybutyrate-co-3-hydroxyhexanoate) (P3HB-3HH), poly(3-hydroxyoctanoate) (P3HO), and poly(3-hydroxybutyrate-co-3-hydroxyoctanoate) (P3HB-3HO).

9. The implant of claim 1, wherein the second biodegradable polymer is one, or a mixture of two or more, selected from synthetic biodegradable polymers and natural biodegradable polymers excluding polyhydroxyalkanoate (PHA),
the synthetic biodegradable polymer comprises polylactic acid (PLA), polycaprolactone (PCL), polyglycolic acid (PGA), poly(lactic acid-co-caprolactone) (PLCL), poly(lactic acid-co-glycolic acid) (PLGA), polybutylene succinate (PBS), or poly(butylene adipate-co-terephthalate) (PBAT), and
the natural biodegradable polymer comprises starch, silk fibroin, chitosan, chitin, cellulose, collagen, or gelatin.

10. The implant of claim 1, wherein the copolymeric polyhydroxyalkanoate (PHA) is included in an amount of 10% by weight to 50% by weight based on the total weight of the first biodegradable polymer and the second biodegradable polymer.

11. The implant of claim 1, wherein the implant further comprises at least one additive selected from bioactive glass fiber or ceramic.

12. The implant of claim 1, wherein the implant further comprises at least one drug for enhancing bone regeneration, and
the drug is coated on the surface of the implant, is blended with the first biodegradable polymer and the second biodegradable polymer, or is included in a concentration gradient from the surface to the inside of the implant.

13. The implant of claim 1, wherein the implant has a tensile strength of 40 MPa to 70 MPa and an elongation of 3.2% to 10%, as measured on specimens prepared with a diameter of 1.5 mm and a length of 50 mm.

14. The implant of claim 1, wherein the implant has a surface roughness with a depth of 1 µm to 10 µm, or a grid pattern, a wave pattern, or a stripe pattern with an interval of 1 µm to 10 µm.

15. The implant of claim 1, wherein the implant, in a cytotoxicity test using an eluate, exhibits a cell viability of 70% or more relative to the cell viability of 100% of an elution solvent control group.

16. The implant of claim 1, wherein when the implant is implanted into a fractured femur tissue of a rat, the stiffness of the femur tissue harvested after 2 months from the implantation is 120 N/mm or more.

17. A process for manufacturing an implant, which comprises:
preparing a biodegradable polymer comprising a first biodegradable polymer and a second biodegradable polymer; and
forming an implant using the biodegradable polymer,
wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).

18. The process for manufacturing an implant of claim 17, wherein the forming is injection molding or 3D printing molding.

19. The process for manufacturing an implant of claim 18, wherein the injection molding is carried out under the conditions of an extrusion temperature of 100°C to 210°C, an injection temperature of 150°C to 200°C, and an annealing temperature of 80°C to 110°C.

20. The process for manufacturing an implant of claim 18, wherein the 3D printing molding is carried out under the conditions of a printing temperature of 150°C to 200°C, an injection speed of 200 mm/minute to 400 mm/minute, a pressure of 200 kPa to 400 kPa, and a printing duration time of 60 minutes or less.

21. The process for manufacturing an implant of claim 17, which further comprises, after the forming, at least one surface treatment step among imparting a roughness or a pattern to the surface through blasting, molding, or laser treatment; and imparting hydrophilicity to the surface through plasma treatment.

22. A composition for an implant, which comprises a first biodegradable polymer and a second biodegradable polymer, wherein the first biodegradable polymer comprises a copolymeric polyhydroxyalkanoate (PHA), and the copolymeric polyhydroxyalkanoate (PHA) comprises a repeat unit derived from 4-hydroxybutyrate (4-HB) in an amount of 0.1% by weight to 50% by weight based on the total weight of the copolymeric polyhydroxyalkanoate (PHA).
